# EUROPÄISCHE PATENTSCHRIFT

(11) **EP 2 310 897 B1**
(45) Veröffentlichungstag und Bekanntmachung des Hinweises auf die Patenterteilung: **05.10.2016**
(21) Anmeldenummer: 09781392.7
(22) Anmeldetag: 03.08.2009
(51) Int. Cl.: G02B 13/22, G02B 23/24, G06T 5/00, A61B 1/00, A61B 1/06, A61B 1/247, G02B 27/00

(54) **DENTALKAMERA**
DENTAL CAMERA
CAMÉRA À USAGE DENTAIRE

(30) Priorität: 01.08.2008 DE 102008040944
(43) Veröffentlichungstag der Anmeldung: 20.04.2011
(73) Patentinhaber: Sirona Dental Systems GmbH, 64625 Bensheim (DE)
(72) Erfinder: SCHMIDT, Volker, 12209 Berlin (DE)
(74) Vertreter: Sommer, Peter
(86) Internationale Anmeldenummer: PCT/EP2009/059994
(87) Internationale Veröffentlichungsnummer: WO 2010/012839

(56) Entgegenhaltungen:
- EP-A- 0 215 566
- EP-A- 0 615 721
- DE-A1- 2 340 687
- DE-A1- 10 316 416
- DE-A1-102005 043 402
- US-A1- 2008 174 678
- US-B1- 6 285 799
- ROGERS J D ET AL: "REMOVAL OF GHOST IMAGES BY USING TILTED ELEMENT OPTICAL SYSTEMS WITH POLYNOMIAL SURFACES FOR ABERRATION COMPENSATION" OPTICS LETTERS, OSA, OPTICAL SOCIETY OF AMERICA, WASHINGTON, DC, US, Bd. 31, Nr. 4, 15. Februar 2006 (2006-02-15), Seiten 504-506, XP001239076 ISSN: 0146-9592
- JOHN R ROGERS: "Techniques and tools for obtaining symmetrical performance from tilted-component systems" OPTICAL ENGINEERING, Bd. 39, Nr. 7, 1. Juli 2000 (2000-07-01), Seiten 1776-1787, XP002551020

## Beschreibung

### Technisches Gebiet

Die Erfindung betrifft eine Dentalkamera mit einem Objektiv und ein Verfahren zur Planung einer solchen Dentalkamera. Das Objektiv umfasst mindestens zwei Linsen, wobei die Dentalkamera eine Lichtquelle, einen Bildsensor und eine Telezentrieblende umfasst, wobei ein von der Lichtquelle ausgestrahlter Beleuchtungsstrahl durch das Objektiv fokussiert wird, auf einem zu vermessenden Messobjekt abgebildet wird und als ein Beobachtungsstrahl vom Messobjekt zurückgestrahlt wird. Der Beobachtungsstrahl durchstrahlt das Objektiv und wird zum Bildsensor gelenkt, wobei der Beleuchtungsstrahl teilweise von Oberflächen einer der Linsen als ein Reflektionsstrahl bildsensorseitig reflektiert wird.

### Stand der Technik

Aus dem Stand der Technik sind mehrere Objektive mit vermindertem Streulicht bekannt. Insbesondere in der Fotografie wird angestrebt das Streulicht, das durch diffuse oder gerichtete Reflektionen zum Bildsensor hin an den äußeren und inneren Oberflächen des Objektivs entsteht, zu vermindern.

Streulichtblenden vermindern das Entstehen von Streulicht, indem seitlicher Lichteinfall in die Optik vermieden wird.

Darüber hinaus werden Oberflächen von Linsen vergütet, um eine gerichtete Reflektion zu vermindern. Insbesondere telezentrische Optiken haben die Eigenschaft, dass das von der Feldlinse zurückgestrahltes Licht auf dem optischen Sensor fokussiert wird.

DE 103 16 416 offenbart ein optisches System für eine Funduskamera, bei welchem zur Vermeidung von Reflexen Linsenpaare gegen den Abbildungsstrahlengang verkippt sind, wobei diese Verkippungen in zwei Ebenen erfolgen und diese Ebenen vorzugsweise senkrecht zueinander orientiert sind. In einer bevorzugten Weiterbildung sind die Linsen in zwei Linsenpaare aufgeteilt, wobei das erste Linsenpaar in einer ersten Ebene und das zweite Linsenpaar in einer zweiten Ebene verkippt sind. In DE 103 16 416 ist eine Lösung aus der US 4,730,910 offenbart, bei der die Linsen so gegeneinander verkippt sind, dass die direkten Reflexe nicht in die Apertur der Beobachtung gelangen.

DE 23 40 687 offenbart eine optische Anordnung mit einer Lichtquelle und einer Linse, wobei die zur Lichtquelle zugewandte Fläche derart gekrümmt ist, dass sie ein reelles Spiegelbild der Lichtquelle entwirft, welches durch die Kippung um einen kleinen Winkel an eine Stelle verlegt wird, wo es am wenigsten stört. Vorteilhafterweise kann an die Stelle des Spiegelbildes eine lichtabsorbierende Fläche gebracht sein. Bei einer weiteren Ausführungsform ist der Krümmungsradius der Fläche annähernd gleich dem Abstand der Fläche von der Lichtquelle. Die Lichtquelle kann auch eine beleuchtete Blendenerfindung sein.

XP-001239076, Optics Letters, Vol. 31, No. 4 offenbart auf Seite 504 ein optisches System bei dem Beleuchtungsstrahlen wie in der Abbildung (a) an der ersten Linsenfläche und wie in Abbildung (b) an der dritten Linsenfläche seitlich reflektiert werden, wobei eine Fokussierung der Reflexionsstrahlen nicht ersichtlich ist.

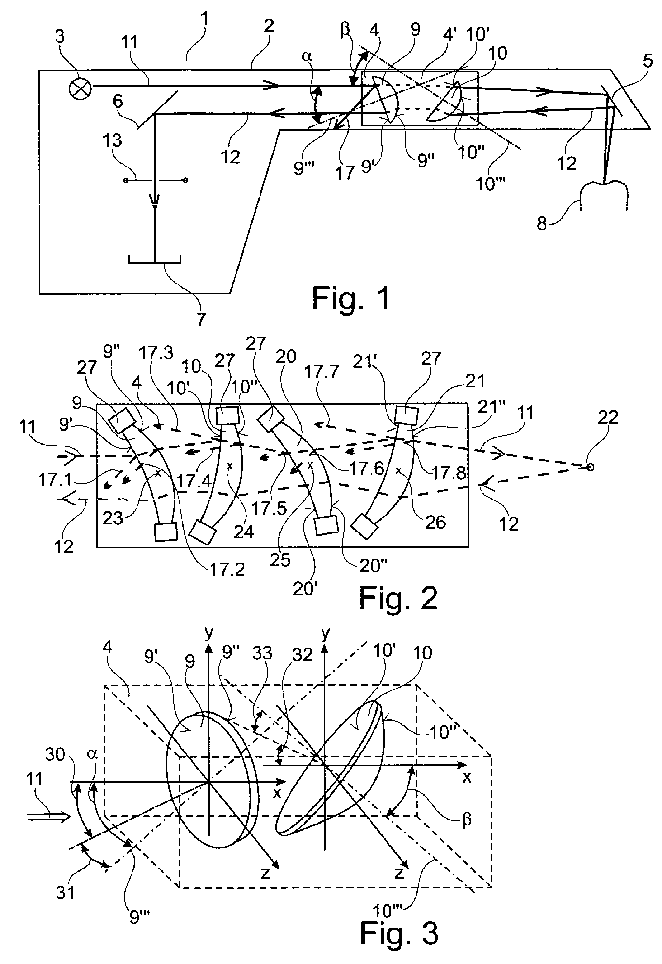

In EP 0 215 566 und EP 0 615 721 ist jeweils eine Kamera für ophthalmologische Anwendungen offenbart. In EP 0 215 566 ist eine Anordnung von gekippten Linsen gezeigt, wobei die Linsenflächen sowohl konkav als auch konvex sind. Aus der Figurenbeschreibung zu Fig. 2E folgt keine besondere Ausgestaltung der Linsenflächen zur Vermeidung der direkten Reflexe. Erfindungsgegenstand ist vielmehr Linsen paarweise anzuordnen, wobei bei einem ersten Paar ein oberer Teil des Linsen und beim zweiten Paar von Linsen ein unterer Teil der Linsen unterhalb der Symmetrieachse zur Beugung der Beleuchtungsstrahlen genutzt wird.

In EP 0 615 721 ist ein Laserbestrahlungssystem offenbart, das vibrierende Linsen aufweist, die zum Bestrahlungsstrahl verkippt sind.

In US 2008/0174678 ist eine Bildbearbeitungssoftware zur digitalen Korrektur von Abbildungsfehlern offenbart, insbesondere zur Korrektur der sphärischen Aberration, Koma, Astigmatismus, Bildkrümmung und Verzerrung.

In US 6,285,799 ist ebenfalls ein Verfahren zur Kalibrierung von Bilddaten einer Kamera offenbart. Die Kalibrierung wird jedoch unter der Verwendung der sogenannten Point spread function (PSF) durchgeführt, um das Hintergrundrauschen der Bilddaten zu verringern.

Die Aufgabe dieser Erfindung besteht darin, eine Dentalkamera mit einem Objektiv bereitzustellen, dessen Abbildungsfehler durch gerichtete Reflektionen an Oberflächen von Linsen des Objektivs vermindert wird.

### Darstellung der Erfindung

Diese Aufgabe wird durch die vorliegende Erfindung gelöst, welche durch die angehängten Ansprüche definiert ist.

Erfindungsgemäß umfasst eine Dentalkamera mit einem Objektiv mindestens zwei Linsen, wobei die Dentalkamera eine Lichtquelle, einen Bildsensor und eine in einer Brennebene des Objektivs angeordnete Telezentrieblende umfasst. Ein von der Lichtquelle ausgestrahlter Beleuchtungsstrahl wird durch das Objektiv fokussiert, auf einen zu vermessende Messobjekt abgebildet und als Beobachtungsstrahl vom Messobjekt zurückgestrahlt. Der Beobachtungsstrahl durchstrahlt das Objektiv und die Telezentrieblende und wird auf den Bildsensor gelenkt, wobei der Beleuchtungsstrahl teilweise von konkaven Oberflächen der Linsen als Reflektionsstrahlen reflektiert wird. Es sind Mittel vorhanden, um jede der Linsen zum Beleuchtungsstrahl hin so zu verkippen, dass eine optische Achse jeder Linse zum Beleuchtungsstrahl einen Verkippungswinkel bildet, der so groß gewählt ist, dass die Reflektionsstrahlen der konkaven Oberflächen auf Fokuspunkte in eine Richtung außerhalb einer Blendenöffnung der Telezentrieblende reflektiert werden.

Durch Abbildung einer Blende im Strahlengang des Beobachtungsstrahls werden die Reflektionsstrahlen somit in eine Richtung außerhalb des Beobachtungsstrahls, der zum Bildsensor gelangt, reflektiert, so dass Reflexeffekte der Linsen verhindert werden. Die Erfindung stellt somit eine Dentalkamera mit Objektiv bereit, bei dem die Reflexeffekte der Linsen durch gezielte Verkippung vermieden werden und die dadurch erzeugten Aberrationen ausgeglichen werden.

Die Gruppe von Linsen des Objektivs, die objektseitig angeordnet ist, wird auch als eine Feldlinse bezeichnet. Die einzelnen Linsen wirken so zusammen, dass die Feldlinse die Beobachtungsstrahlen auf die Ebene des Messobjekts fokussiert.

Das Objektiv weist Mittel auf, um die Linsen zum Beleuchtungsstrahl zu verkippen. Diese Mittel können Haltevorrichtungen, wie Halteringe oder Haltefassungen für Linsen sein, die die Linsen in einer bei der Planung des Objektivs vorbestimmten Lage mit einem bestimmten Winkel zum Beobachtungsstrahl positionieren. Dabei ist es vorteilhaft die Linsen schon bei der Montage mit einem definierten Kippwinkel einzubauen.

Eine dentale Kamera wird intraoral eingesetzt, um Strukturen wie Zähne oder Zahnstümpfe als Messobjekt aufzunehmen. Das erfindungsgemäße Objektiv umfasst mindestens zwei Linsen, die gegeneinander verkippt sind. Die Lichtquelle sendet einen Beleuchtungsstrahl aus, der abhängig vom verwendeten Messverfahren ein monochromatisches oder ein polychromatisches Spektrum aufweist. Die Linsen weisen optische Achsen auf, die durch den Mittelpunkt der Linsen tretende Symmetrieachsen darstellen. Die Linsen sind so verkippt, dass von beiden Oberflächen jeder Linse, nämlich einer zur Lichtquelle hin zeigenden ersten Oberfläche und einer von der Lichtquelle weg zeigenden zweiten Oberfläche, Reflektionsstrahlen in eine Richtung außerhalb des Strahlengangs des Beobachtungsstrahls reflektiert werden, so dass es zu keinem störenden Reflexeffekt durch Überlagerung des Reflektionsstrahles und des Beobachtungsstrahls kommt. Die Reflektionsstrahlen treffen somit insbesondere nicht auf den Bildsensor, so dass durch reflektiertes Licht verursachte Bildstörungen vermieden werden.

Als Beleuchtungsart kann die sogenannte Köhlersche Beleuchtung verwendet werden, die vor allem für optische Aufbauten in der Mikroskopie verwendet wird.

Die Köhlersche Beleuchtung soll eine möglichst helle und gleichmäßige Beleuchtung des Messobjekts garantieren.

Durch eine Kollektorlinse wird das Licht der Lichtquelle gebündelt. Die Lichtquelle selbst wird durch eine Kollektorlinse in der Ebene einer sogenannten Aperturblende abgebildet, die in einer Brennebene der Kondensorlinse angeordnet ist. Durch die Kondensorlinse wird dieses Bild der Lichtquelle als gleichmäßig verteiltes Lichtfeld auf die Ebene des Messobjektes abgebildet. Die Beleuchtungsstrahlen fallen durch die Kondensorlinse als parallele Strahlenbündel auf das Messobjekt und bilden ein Beleuchtungsfeld mit einer gleichmäßigen Leuchtstärke.

Das Objektiv gemäß der Erfindung ist insbesondere zur Anwendung eines sogenannten Triangulationsverfahrens zur dreidimensionalen Vermessung geeignet.

Ein Triangulationsverfahren ist zur optischen Vermessung von dreidimensionalen Geometrien eines Messobjekts geeignet, wobei auf das aufzunehmende Messobjekt ein Muster projiziert wird, das mehrere parallele Lichtstreifen aufweisen kann. Das projizierte Muster wird in mehreren Aufnahmen aufgenommen. Aus dem Verlauf der Projektionen des produzierten Musters kann dann auf die dreidimensionale Geometrie des Messobjekts zurückgerechnet werden.

Durch die Verminderung des Reflexeffekts wird das Bild des Messobjekts nicht durch punktuelle Aufhellung verfälscht.

Darüber hinaus ist beim Objektiv der erfindungsgemäßen Dentalkamera keine aufwendige Vergütung der Linsenoberflächen zur Reduktion der

Reflektionen notwendig. Eine zusätzliche Vergütung der Linsen kann jedoch die optischen Eigenschaften verbessern.

Vorteilhafterweise kann mindestens eine konkave Oberfläche der Linsen eine solche Krümmung aufweisen, dass der Reflektionsstrahl an einem Fokuspunkt fokussiert wird, der in der Ebene einer Telezentrieblende und außerhalb der Blendenöffnung liegt.

Die konkave Form kann eine sphärische Krümmung oder eine sonstige Krümmung aufweisen, die so geformt ist, dass die einfallenden Beleuchtungsstrahlen durch Reflektion zurückgestrahlt werden und die Reflektionsstrahlen am Fokuspunkt fokussiert werden.

Die Beleuchtungsstrahlen treffen zunächst auf die lichtquelleseitige konkave Oberfläche der ersten lichtquelleseitigen Linse auf und dann auf die andere von der Lichtquelle abgewandte Seite der ersten Linsen auf. Die Reflektionsstrahlen von der lichtquelleseitigen Oberfläche werden dann auf einen ersten Fokuspunkt fokussiert. Die Reflektionsstrahlen von der objektseitigen Oberfläche werden zunächst an der lichtquelleseitigen Oberfläche gebrochen und in einem zweiten Fokuspunkt fokussiert. Die Reflektionsstrahlen der lichtquelleseitigen Oberflächen und der von der Lichtquelle abgewandten Oberflächen der übrigen Linsen, die zur Feldlinse gehören, werden durch die Oberflächen der Linsen die zwischen ihnen und der Lichtquelle liegen gebrochen und ebenfalls auf die jeweiligen Fokuspunkte fokussiert. Die unterschiedlichen Fokuspunkte, die außerhalb der Blendenöffnung liegen, können an einem gemeinsamen Punkt angeordnet sein oder einen Abstand zueinander aufweisen.

Dadurch reicht schon eine geringe Verkippung der Linsen aus, um die auf die jeweiligen Fokuspunkte fokussierten Reflektionsstrahlen auf einen Bereich außerhalb der Blendenöffnung der Telezentrieblende zu lenken. Die notwendige Verkippung der Linsen hängt von der Apertur des Abbildungssystems ab.

Vorteilhafterweise kann eine zweiten Linse um einen Verkippungswinkel β gegenüber dem Beleuchtungsstrahl verkippt werden, wobei die zweite Linse der ersten Linse in Richtung des Beleuchtungsstrahls nachgeschaltet ist und dass die durch die Verkippung der ersten Linse um einen Winkel α verursachten Aberrationen durch eine zweite Linse ganz oder teilweise kompensiert werden, so dass die gewünschten optischen Eigenschaften des Objektivs erreicht werden.

Dadurch wirkt das gesamte optische System aus der ersten und der zweiten Linse, die zusammen die Feldlinse bilden, wie eine herkömmliche Feldlinse mit gewünschten optischen Eigenschaften, die aus einer einzelnen Linse oder aus mehreren parallel zueinander angeordneten Linsen besteht. Bei der Kompensation der Abberationen kann allerdings auch ein Kompensationsrestfehler auftreten.

Vorteilhafterweise sind die geometrischen Parameter und die Materialeigenschaften, wie Radien, Glassorten, Brechungsindizes, Abstände und Verkippungswinkel der Linsen so gewählt, dass die Aberrationen hinreichend kompensiert werden, um die gewünschten optischen Eigenschaften des Objektivs zu erreichen.

Dadurch wird die Kompensation optimiert, so dass der Kompensationsrestfehler vermindert wird. Die Wahl der Parameter erfolgt nach üblichen Auswahlkriterien, um die optischen Abbildungsfehler, wie Aberrationen, Koma, Astigmatismus, Distorsion, chromatische Aberration, Streulicht und Absorption zu minimieren.

Die Wahl der geometrischen Parameter und der Materialeigenschaften wie Radien, der Glassorten, der Brechungsindizes, der Abstände und der Verkippungswinkel der Linsen kann beispielsweise unter Verwendung eines Computers mittels eines Optimierungsalgorithmus erfolgen.

Mittels eines Optimierungsalgorithmus werden die Parameter für eine optimale Kompensation berechnet, so dass der Kompensationsrestfehler minimiert wird.

Vorteilhafterweise kann die Kompensation der Aberrationen bei einer geraden Anzahl von Linsen paarweise erfolgen.

Dadurch wird die Bestimmung der einzelnen Linsenparameter vereinfacht, da jeweils nur Parameter von zwei Linsen berücksichtigt werden müssen. Darüber hinaus ist die Berechnung der maßgeblichen Linsenparameter bei einer Kompensation eines Linsenpaars weniger aufwendig als einer Kompensation einer Linsengruppe aus drei oder mehr Linsen.

Die Verkippung der ersten lichtquellseitig angeordneten Linse kann beispielsweise um einen Winkel α in zwei Ebenen erfolgen, nämlich durch eine erste Verkippung in einer ersten Ebene und eine zweite Verkippung in einer zweiten Ebene.

Dadurch kann der Reflektionsstrahl in eine Richtung gerichtet werden, die einen größeren Winkel zum Strahlengang des Beobachtungsstrahls aufweist und somit den Reflexeffekt vermindert. Die beiden Ebenen können insbesondere senkrecht zueinander sein.

Vorteilhafterweise kann die Verkippung der ersten lichtquellseitig angeordneten Linse um eine Verkippungsachse erfolgen, wobei die übrigen Linsen ebenfalls um ihre Verkippungsachsen verkippt werden, die parallel zur Verkippungsachse der ersten Linse sind, wobei die Verkippungsachsen senkrecht zur Triangulationsebene sind.

Die Verkippungsachsen sind parallel zueinander angeordnet, so dass die optischen Achsen der Linsen in einer Ebene liegen.

Die Verkippungsachsen stellen Achsen dar, um die die einzelnen Linsen relativ zur Ausgangslage, in der die optische Achse parallel zur Ausrichtung des Beleuchtungsstrahls ausgerichtet ist, verkippt sind.

Dadurch wird die Bestimmung der Linsenparameter erleichtert, da die Freiheitsgrade der Verkippung durch die Bedingung der parallelen Verkippungsachsen eingeschränkt werden.

Die Triangulationsebene wird durch den Beleuchtungsstrahl und den Beobachtungsstrahl aufgespannt. Die Verkippungsachsen sind senkrecht zur Triangulationsebene und parallel zueinander, so dass die Verkippungsachsen nicht in einer Ebene liegen müssen und die Linsen versetzt zueinander angeordnet sein können.

Es kann beispielsweise eine Kalibriereinheit vorhanden sein, wobei ein Kompensationsrestfehler, der bei der optischen Kompensation der Verkippungen der Linsen entsteht, als ein systematischer Fehler mittels der Kalibriereinheit durch ein Kalibrierverfahren aus den Bilddaten des Bildsensors herausrechenbar ist.

Dadurch wird der Kompensationsrestfehler als ein systematischer Fehler korrigiert und die Abbildungsgenauigkeit verbessert.

Ein weiterer Gegenstand der Erfindung ist ein Verfahren zur Planung einer Dentalkamera mit einem Objektiv, wobei das Objektiv mindestens zwei Linsen umfasst, wobei die Dentalkamera eine Lichtquelle, einen Bildsensor und eine in einer Brennebene des Objektivs angeordnete Telezentrieblende umfasst, wobei ein von der Lichtquelle ausgestrahlter Beleuchtungsstrahl durch das Objektiv fokussiert wird, auf ein zu vermessendes Messobjekt abgebildet wird und als ein Beobachtungsstrahl vom Messobjekt zurückgestrahlt wird, wobei der Beobachtungsstrahl das Objektiv und die Telezentrieblende durchstrahlt und auf den Bildsensor gelenkt wird, wobei der Beleuchtungsstrahl teilweise von konkaven Oberflächen der Linsen als Reflektionsstrahlen reflektiert wird. Jede der Linsen wird zum Beleuchtungsstrahl bei der Planung so verkippt, dass eine optische Achse jeder Linse einen Verkippungswinkel zum Beleuchtungsstrahl hin bildet, der so groß gewählt ist, dass die Reflektionsstrahlen in eine Richtung außerhalb einer Blendenöffnung der Telezentrieblende reflektiert werden.

Das erfinderische Verfahren kann unter Verwendung des vorbeschriebenen Objektivs durchgeführt werden.

Ein Vorteil dieses Verfahrens ist, dass die Linsen nicht wie herkömmlich in einem aufwendigen Verfahren veredelt werden müssen, um den Reflexeffekt zu vermindern, sondern durch die Verkippung der einzelnen Linsen die Reflektionsstrahlen in eine Richtung außerhalb der Pupille des Beobachtungsstrahls reflektiert werden und somit nicht zum Bildsensor gelangen. Vergütete Linsen zeigen allerdings ohne Verkippung trotz geringerer Reflexionen verminderte Reflexeffekte, da ein geringer Teil des Lichts reflektiert wird. Bei einer Verkippung der einzelnen Linsen kann der Reflexeffekt nahezu ausgeschlossen werden.

Vorteilhafterweise können bei der Planung der Dentalkamera mit Objektiv die geometrischen Parameter und die Materialeigenschaften, wie Radien, Glassorten, Brechungsindizes, Abstände und Verkippungswinkel der Linsen so gewählt werden, dass die Aberrationen hinreichend kompensiert werden, um die gewünschten optischen Eigenschaften des Objektivs zu erreichen.

Die Wahl der geometrischen Parameter und die Materialeigenschaften der Linsen bei der Planung des Objektivs ist maßgeblich für den Grad der Kompensation der Abbildungsfehler.

Vorteilhafterweise kann die Wahl der geometrischen Parameter und der Materialeigenschaften, wie der Radien, der Glassorten, der Brechungsindizes, der Abstände und der Verkippungswinkel der Linsen unter Verwendung eines Computers mittels eines Optimierungsalgorithmus erfolgen.

Dadurch wird die Zeitdauer der Planung verkürzt und die computergestützte Wahl der geometrischen Parameter und der Materialeigenschaften führt zu einer computergestützten Optimierung der Kompensation bei einer Verringerung des Kompensationsrestfehlers.

Vorteilhafterweise kann bei der Kompensation ein systematischer Kompensationsrestfehler entstehen, der mittels eines Kalibrierungsverfahrens unter Verwendung einer Kalibriereinheit aus den Bilddaten des Bildsensors herausgerechnet wird.

Dadurch wird der Kompensationsrestfehler kalibriert und die optische Abbildungsqualität verbessert.

Vorteilhafterweise können die dem Objektiv vorgeschalteten und nachgeschalteten optischen Bauteile mittels eines Optimierungsalgorithmus unter Verwendung eines Computers angepasst werden.

Falls das beschriebene Objektiv in ein bestehendes System mit weiteren optischen Bauteilen eingesetzt wird, können die übrigen optischen Bauteile mittels eines Optimierungsalgorithmus an die veränderten optischen Eigenschaften des Objektivs angepasst werden.

### Kurzbeschreibung der Zeichnungen

Ausführungsbeispiele der Erfindung sind in den Zeichnungen dargestellt. Es zeigt die
- Fig. 1: eine erfindungsgemäße Dentalkamera mit einem Objektiv bestehend aus zwei Linsen;
- Fig. 2: das Objektiv der Dentalkamera mit vier Linsen;
- Fig. 3: das Objektiv der Dentalkamera mit zwei Linsen, wobei die Kompensation in zwei Ebenen erfolgt;
- Fig. 4: das Objektiv der Dentalkamera mit zwei Linsen und zwei Achromaten;
- Fig. 5: ein Schema zur Erläuterung der Kalibrierung;
- Fig. 6: eine alternative Ausführungsform der erfindungsgemäßen Dentalkamera mit Objektiv.

### Ausführungsbeispiel

Die Fig. 1 zeigt eine Dentalkamera 1 mit einem Kameragehäuse 2, umfassend eine Lichtquelle 3, ein Objektiv 4, einen ersten Umlenkspiegel 5, einen zweiten Umlenkspiegel 6 und einen Bildsensor 7. Die dargestellte Dentalkamera eignet sich zur intraoralen Anwendung, um intraorale Strukturen, wie beispielsweise einen dargestellten Zahn, als ein Messobjekt 8 aufzunehmen. Das Objektiv 4 weist in der dargestellten Ausführungsform zwei gegeneinander verkippten Linsen 9 und 10 auf, die im Objektiv 4 in einer vorbestimmten Weise an einer geplanten Position mit einem geplanten Winkel angeordnet sind. Die Linsen 9 und 10 bilden eine so genannte Feldlinse 4'. Die Lichtquelle 3 sendet einen Beleuchtungsstrahl 11 aus, der innerhalb des Objektivs 4 durch die Linsen 9 und 10 fokussiert wird, durch den ersten Umlenkspiegel 5 auf das Messobjekt 8 umgelenkt wird und vom Messobjekt 8 als ein Beobachtungsstrahl 12 zurückgestrahlt wird und als Beobachtungsstrahl 12 vom ersten Umlenkspiegel 5 zurück zum Objektiv 4 umgelenkt wird. Der Beobachtungsstrahl 12 wird vom Objektiv weiter zum zweiten Umlenkspiegel 6 geleitet und vom zweiten Umlenkspiegel 6 zum Bildsensor 7 umgelenkt, der die Bilddaten zur weiteren Auswertung liefert.

Zwischen dem zweiten Umlenkspiegel 6 und dem Bildsensor 7 ist eine Telezentrierblende 13 in der Brennebene des Objektivs 4 angeordnet, so dass durch die Telezentrierebene nur diejenigen Strahlen durchgelassen werden, die durch den Brennpunkt des Objektivs 4 laufen. Diese telezentrische Anordnung hat den Vorteil, dass bei Änderung der Gegenstandsweite der Abbildungsmaßstab und die Tiefenschärfe nahezu konstant bleiben.

Ein Nachteil der telezentrischen optischen Anwendung ist die geringe Lichtstärke des am Bildsensor 7 ankommenden Lichtsignals. Eine Voraussetzung für die Funktionsweise des telezentrischen Objektivs ist, dass der Durchmesser des Objektivs zumindest der Objektgröße des Messobjekts 8 entsprechen muss.

Darüber hinaus wird als Beleuchtungsart die sogenannte Köhlersche Beleuchtung verwendet, die vor allem für Optiken in der Mikroskopie verwendet wird.

Die Köhlersche Beleuchtung soll eine möglichst helle und gleichmäßige Beleuchtung des Messobjekts gewährleisten.

Durch eine nicht dargestellte Kollektorlinse wird das Licht der Lichtquelle gebündelt. Die Lichtquelle selbst wird durch eine Kollektorlinse in der Ebene einer sogenannten nicht dargestellten Aperturblende, die der Telezentrieblende entspricht, abgebildet, die sich in der Brennebene der Feldlinse 4' befindet. Das gebündelte Bild der Lichtquelle wird dadurch als gleichmäßig verteiltes Licht durch die Feldlinse 4' auf die Ebene des Messobjektes 8 abgebildet. Dadurch weist ein Beleuchtungsfeld der Beleuchtungsstrahlen 11 eine gleichmäßig verteilte Leuchtstärke auf.

An der Stelle des zweiten Umlenkspiegels 6 kann auch ein halbdurchlässiger Strahlteiler angeordnet sein, der den Beleuchtungsstrahl teilweise durchlässt und den Beobachtungsstrahl 12 zum Bildsensor 7 umlenkt. Dabei stimmt der Strahlengang des Beleuchtungsstrahls 11 mit dem Strahlengang des Beleuchtungsstrahls 12 zwischen dem Strahlteiler und dem Messobjekt 8 überein.

Die Lichtquelle 3 kann abhängig vom verwendeten Aufnahmeverfahren eine polychromatische oder monochromatische Lichtquelle sein. Die Linse 9 weist eine optische Achse 9"' und die Linse 10 weist eine optische Achse 10''' auf. Die erste Linse 9, die lichtquellenseitig angeordnet ist, ist um einen Winkel α so stark gegenüber dem Beleuchtungsstrahl 11 verkippt, dass ein von der lichtquellenseitigen Oberfläche 9' der ersten Linse 9 ausgehende Reflektionsstrahl 17 in eine Richtung außerhalb des Strahlengangs des Beobachtungsstrahlengangs 12 reflektiert wird, so dass es auf dem Bildsensor zu keinem störendem Reflexeffekt durch eine Überlagerung des Reflektionsstrahles 17 und des Beobachtungsstrahls 12 kommt. Durch die Verminderung des Reflexeffekts wird der Bildkontrast erhöht und das Bild wird nicht durch punktuelle Aufhellung verfälscht.

Die zweite Linse 10 ist relativ zur ersten Linse 9 so angeordnet, dass die durch die Verkippung der ersten Linse 9 entstehenden Aberrationen durch die zweite Linse 10 wieder weitestgehend kompensiert werden. Das gesamte Objektiv 4 bestehend aus der Linse 9 und 10 weist als optisches System die gewünschten optischen Eigenschaften eines herkömmlichen Objektivs auf. Zu den gewünschten optischen Eigenschaften gehört eine Korrektion der Fehler wie der sphärischen Aberration, der Bildfeldwölbung, des Astigmatismus, der Distorsion und chromatischen Aberration. Die Radien, die Glassorten, die Brechungsindizes, die Abstände und die Verkippungswinkel der Linsen 9 und 10 werden so gewählt, dass die gewünschten optischen Eigenschaften des Objektivs 4 erreicht werden. Die Optimierung dieser Parameter kann mittels eines Optimierungsalgorithmus unter Verwendung eines Computers durchgeführt werden.

Eine objektseitige Oberfläche 9" der ersten Linse 9, eine lichtquellenseitige Oberfläche 10' und eine objektseitige Oberfläche 10'' der zweiten Linse 10 sind ebenfalls so angeordnet, dass die Reflektionsstrahlen von diesen Oberflächen nicht auf den Bildsensor 7 treffen.

Eine Triangulationsebene 28 wird durch den Beleuchtungsstrahl 11 und den Beobachtungsstrahl 12 aufgespannt.

Die Fig. 2 zeigt das Objektiv 4 der erfindungsgemäßen Dentalkamera aus Fig. 1 mit vier Linsen 9, 10, 20 und 21. Der Strahlengang des Beleuchtungsstrahls 11 und der Strahlengang des Beobachtungsstrahls 12 sind schematisch aufgezeigt. Die Kompensation der Aberrationen erfolgt paarweise, so dass die durch die Verkippung der ersten Linse 9 entstehenden Aberrationen durch die Verkippung der zweiten Linse 10 kompensiert werden und die durch die Verkippung der dritten Linse 20 entstehenden Aberrationen durch die Verkippung der vierten Linse 21 kompensiert werden. Die Feldlinse 4' des Objektivs 4 weist optischen Eigenschaften eines herkömmlichen Objektivs mit einem punktförmigen Brennpunkt 22 auf. Die Linsen 9, 10, 20 und 21 sind um die Verkippungsachsen 23, 24, 25 und 26 verkippt, wobei die Verkippungsachsen 23, 24, 25 und 26 parallel zueinander und in eine Ebene angeordnet sind, so dass die optischen Achsen 9'", 10'", 20'" und 21"' der Linsen 9, 10, 20 und 21 in einer Ebene verkippt sind. Das Objektiv 4 kann auch fünf oder mehr Linsen aufweisen.

Die Linsen 9, 10, 20 und 21 sind so verkippt, dass der Reflektionsstrahl 17.1 von der lichtquelleseitigen Oberfläche 9', der Reflektionsstrahl 17.2 von der objektseitigen Oberfläche 9", der Reflektionsstrahl 17.3 von der lichtquelleseitigen Oberfläche 10', der Reflektionsstrahl 17.4 von der objektseitigen Oberfläche 10", der Reflektionsstrahl 17.5 von der lichtquelleseitigen Oberfläche 20', der Reflektionsstrahl 17.6 von der objektseitigen Oberfläche 20", der Reflektionsstrahl 17.7 von der lichtquelleseitigen Oberfläche 21' und der Reflektionsstrahl 17.8 von der objektseitigen Oberfläche 21" in eine Richtung außerhalb der Blendenöffnung 13.1 der Telezentrieblende 13 abgelenkt werden und somit nicht zum Bildsensor 7 gelangen.

Das Objektiv 4 weist Mittel 27 zur Halterung der Linsen 9, 10, 20 und 21, wie Haltefassungen auf, die eine Lagerung der Linsen 9, 10, 20 und 21 in einer bei einer Planung des gewünschten Objektivs 4 vorbestimmten Weise gewährleisten.

Fig. 3 zeigt das Objektiv 4 aus Fig. 1 in einer räumlichen Ansicht, wobei die erste Linse 9 und die zweite Linse 10 nicht nur in einer Ebene, sondern in zwei Ebenen verkippt sind. Die optische Achse 9'" der Linse 9 und die optische Achse 10'" der Linse 10 sind als Strichpunktlinien dargestellt. Ein kartesisches Koordinatensystem mit den Achsen x, y und z wurde in den Mittelpunkt der Linse 9 gelegt. Die Linse 9 wurde in der xy-Ebene um den Winkel 30 verkippt und zusätzlich in der xz-Ebene um den Winkel 31 verkippt. Der Abstand und die Verkippung der Linse 10 wurden so gewählt, dass die durch die Verkippung der Linse 9 entstehenden Aberrationen kompensiert werden. In den Mittelpunkt der Linse 10 wurde ebenfalls ein kartesisches Koordinatensystem mit den Achsen x, y und z hineingelegt. Die zweite Linse 10 wurde in der xy-Ebene um den Winkel 32 verkippt und zusätzlich in der xz-Ebene um den Winkel 33 verkippt.

Das Objektiv 4 kann auch mehrere Linsen die in zwei oder drei Ebenen verkippt sind aufweisen, wobei jeweils ein Paar von Linsen die durch die Verkippung entstehenden Aberrationen kompensiert.

Die Winkel α und β der Verkippungen der Linsen 9, 10 zum Beleuchtungsstrahl 11 sind so gewählt, dass der Reflektionsstrahl 17.1 von der lichtquelleseitigen Oberfläche 9', der Reflektionsstrahl 17.2 von der objektseitigen Oberfläche 9", der Reflektionsstrahl 17.3 von der lichtquelleseitigen Oberfläche 10' und der Reflektionsstrahl 17.4 der objektseitigen Oberfläche 10" in eine Richtung außerhalb der Blendenöffnung 13.1 der in Fig.1 dargestellten Telezentrieblende 13 abgelenkt werden und somit nicht zum Bildsensor 7 gelangen.

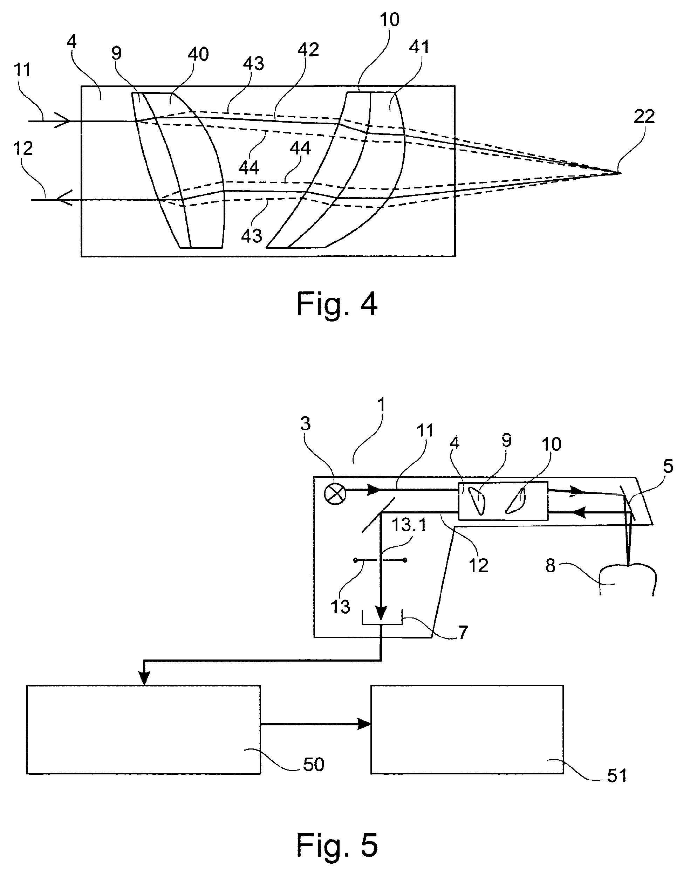

Fig. 4 zeigt schematisch das Objektiv 4 aus Fig. 1 bestehend aus einer ersten Linse 9 und einer zweiten Linse 10, wobei zusätzlich an jeder Linse eine weitere Linse 40 und 41 gleicher Größe angebracht wurde, wobei das optische System aus den Linsen 9, 40, 10 und 41 einen Achromat bilden. Unter einem Achromat versteht man in der Optik ein System aus mehreren Linsen von gleicher Größe, die aus Gläsern verschiedener Abbezahl bestehen. Ein Achromat führt dazu, dass der Abbildungsfehler der chromatischen Aberration korrigiert wird. Ein Achromat aus zwei Linsen kann die chromatische Aberration für zwei Wellenlängen korrigieren, wobei die Form der Linsen und das Verhältnis der Abbezahlen so gewählt wird, dass die Fokuspunkte der Lichtstrahlen der beiden Wellenlängen übereinstimmen. Bei einem Achromaten mit drei Linsen kann die chromatische Aberration für drei Wellenlängen kompensiert werden.

Zusätzlich lässt sich durch geeignete Geometrie- und Materialauswahl der Öffnungsfehler (sphärische Aberration) minimieren, Achromate eigenen sich somit auch hervorragend bei monochromatischer Anwendung zur optimalen Fokussierung. Achromate erlauben auch die Korrektion der Koma im achsnahen Gebiet. Diese Art der Korrektion gewährleistet eine Unempfindlichkeit des optischen Systems gegenüber kleinen Verkippungen. Kleine und ausgedehnte Objekte lassen sich daher gut abbilden.

Im vorliegenden Fall wurde die chromatische Aberration des Objektivs 4 für das rote und blaue Licht kompensiert. Die volle Linie zeigt den Strahlengang des grünen Lichtstrahls 42, eine gestrichelte Linie zeigt den Strahlengang des blauen Lichts 43 und die zweite gestrichelte Linie zeigt den Strahlengang des roten Lichts 44. Es ist der Beleuchtungsstrahl 11 und der Beobachtungsstrahl 12 dargestellt. Die Strahlengänge des blauen Lichts 43, des grünen Lichts 43 und des roten Lichts 44 weisen den gleichen Brennpunkt 22 auf, so dass die chromatische Aberration für die Wellenlängen des blauen, des grünen und des roten Lichts kompensiert wurde.

Fig. 5 zeigt ein Schema zur Verdeutlichung der Kalibrierung eines Kompensationsrestfehlers, der bei der Kompensation der Aberrationen im Objektiv 4 entsteht. Die Radien, die Glassorten, die Brechungsindizes, die Abstände sowie die Verkippungswinkel α, β der Linsen 9 und 10 werden so gewählt, dass die durch die Verkippung der ersten Linse 9 verursachten Aberrationen nahezu vollständig kompensiertwerden. Die Wahl dieser Faktoren kann mittels eines Optimierungsalgorithmus unter Verwendung eines Computers erfolgen. Eine Vollständige Kompensation kann in der Praxis jedoch nicht erreicht werden, so dass ein Kompensationsrestfehler zu einem Abbildungsfehler führt. Dieser Kompensationsrestfehler kann mittels einer Kalibriereinheit 50 kalibriert werden und als systematischer Fehler aus den Bilddaten des Bildsensors 7 herausgerechnet werden. Die korrigierten Bilddaten werden dann an eine Auswerteeinheit 51 zur weiteren Bildanalyse übermittelt.

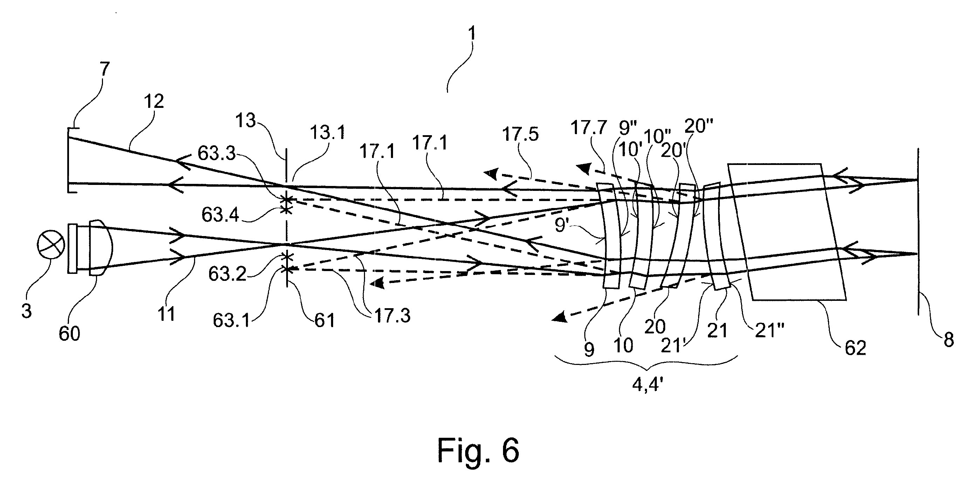

Die Fig. 6 zeigt eine alternative Ausführungsform der Dentalkamera 1, umfassend eine Lichtquelle 3, ein Objektiv 4 und einen Bildsensor 7. Die Lichtquelle 3 sendet einen Beleuchtungsstrahl 11 aus, der durch eine Kollektorlinse 60 gebündelt wird. Die Lichtquelle 7 selbst wird durch die Kollektorlinse 60 in der Ebene einer Aperturblende 61 abgebildet, die sich in der Brennebene der Feldlinse 4', die aus den Linsen 9, 10, 20, 21 besteht, befindet. Das gebündelte Bild der Lichtquelle wird als gleichmäßig verteiltes Licht durch die Feldlinse 4' auf die Ebene des Messobjektes 8 abgebildet. Dadurch weist ein Beleuchtungsfeld der Beleuchtungsstrahlen 11 eine gleichmäßig verteilte Leuchtstärke auf, wobei die Beleuchtungsstrahlen 11 parallel fokussiert auf die Ebene des Messobjekts 8 auftreffen. Diese Beleuchtungsart wird die Köhlersche Beleuchtung genannt.

Zwischen der Feldlinse 4' und der Oberfläche des Messobjekts 8 ist ein Prisma 62 angeordnet, das die Beleuchtungsstrahlen 11 ausrichtet. Die Beleuchtungsstrahlen 11 treffen auf die Oberfläche des Messobjekts 8 und werden als Beobachtungsstrahlen 12 zurückgestrahlt. Die Beobachtungsstrahlen 12 werden vom Prisma 62 und den Linsen 9, 10, 20, 21 gebündelt und auf die Blendenöffnung der Telezentrieblende 13 fokussiert und gelangen zum Bildsensor 7. Die Aperturblende 61 und die Telezentrieblende 13 sind in der gleichen Ebene angeordnet.

Die lichtquellenseitige Oberfläche 9' und die objektseitige Oberfläche 9'' der ersten Linse 9 sowie die lichtquellenseitige Oberfläche 10'und die objektseitige Oberfläche 10'' der zweiten Linse 10 weisen eine konkave Krümmung auf, die so gewählt ist, dass der Reflektionsstrahl 17.1 auf einen ersten Fokuspunkt 63.1 fokussiert wird, dass der Reflektionsstrahl 17.2 auf einen zweiten Fokuspunkte 63.2 fokussiert wird, dass der Reflektionsstrahl 17.3 auf einen dritten Fokuspunkt 63.3 fokussiert wird und dass der Reflektionsstrahl 17.4 auf einen vierten Fokuspunkt 63.4 fokussiert wird. Dabei liegen alle Fokuspunkte 63.1 bis 63.4 in der Ebene der Telezentrieblende 13 und zwar außerhalb der Blendenöffnung 13.1 der Telezentrieblende 13. Die Oberflächen 20', 20'', 21', 21'' der übrigen Linsen 20, 21 weisen keine konkave Krümmung auf, sind jedoch so gekippt, dass die Reflektionsstrahlen 17. 5 bis 17.8 in eine Richtung außerhalb der Blendenöffnung 13.1 der Telezentrieblende 13 reflektiert werden.

## Patentansprüche

1. Dentalkamera (1) mit einem Objektiv (4), umfassend mindestens zwei Linsen (9, 10, 20, 21), wobei die Dentalkamera (1) eine Lichtquelle (3), eine Kollektorlinse (60), einen Bildsensor (7) und eine in einer Brennebene des Objektivs (4) angeordnete Telezentrieblende (13) umfasst, wobei ein von der Lichtquelle (3) ausgestrahlter Beleuchtungsstrahl (11) durch die Kollektorlinse (60) gebündelt wird, indem die Lichtquelle (7) durch die Kollektorlinse (60) in der Ebene einer Aperturblende (61) abgebildet, die sich in der Brennebene des Objektivs (4) befindet, so dass das gebündelte Bild der Lichtquelle (3) als gleichmäßig verteiltes Licht auf die Ebene des zu vermessenden Messobjekts (8) abgebildet wird und als ein Beobachtungsstrahl (12) vom Messobjekt (8) zurückgestrahlt wird, wobei der Beobachtungsstrahl (12) das Objektiv (4) und die Telezentrieblende (13) durchstrahlt und auf den Bildsensor (7) gelenkt wird, wobei der Beleuchtungsstrahl (11) teilweise von konkaven Oberflächen (9', 9'', 10', 10'', 20', 20'') der Linsen (9, 10, 20, 21) als Reflektionsstrahlen (17.1 bis 17.8) reflektiert wird, wobei Mittel (27) vorhanden sind, um jede der Linsen (9, 10, 20, 21) zum Beleuchtungsstrahl (11) hin so zu verkippen, dass eine optische Achse (9''', 10''', 15.1, 15.2) jeder Linse (9, 10, 20, 21) zum Beleuchtungsstrahl (11) einen Verkippungswinkel (α) bildet, der so groß gewählt ist, dass die Reflektionsstrahlen (17.1 bis 17.8) der konkaven Oberflächen (9', 9'', 10', 10'', 20', 20'') auf Fokuspunkte (63.1 bis 63.4) in eine Richtung außerhalb einer Blendenöffnung (13.1) der Telezentrieblende (13) reflektiert werden, wobei mindestens eine konkave Oberfläche (9', 9', 9'', 10', 10'', 20', 20'') der Linsen (9, 10, 20) eine solche Krümmung aufweist, dass der Reflektionsstrahl an einem Fokuspunkt (63.1 bis 63.4) fokussiert wird, der in der Ebene der Telezentrieblende (13) und außerhalb der Blendenöffnung (13.1) der Telezentrieblende (13) liegt.

2. Dentalkamera nach Anspruch 1, worin eine zweiten Linse (10) um einen Verkippungswinkel (β) gegenüber dem Beleuchtungsstrahl (11) verkippt wird, wobei die zweite Linse (10) der ersten Linse in Richtung des Beleuchtungsstrahls (11) nachgeschaltet ist und dass die durch die Verkippung der ersten Linse (9) um einen Winkel (α) verursachten Aberrationen durch eine zweite Linse (10) kompensiert werden, so dass die gewünschten optischen Eigenschaften des Objektivs (4) erreicht werden.

3. Dentalkamera nach einem der Ansprüche 1 bis 2, worin die geometrischen Parameter und die Materialeigenschaften, wie Radien, Glassorten, Brechungsindizes, Abstände und Verkippungswinkel (α, β) der Linsen (9, 10, 20, 21) so gewählt sind, dass die Aberrationen hinreichend kompensiert werden, um die gewünschten optischen Eigenschaften des Objektivs (4) zu erreichen.

4. Dentalkamera nach einem der Ansprüche 1 bis 3, worin die Kompensation der Aberrationen bei einer geraden Anzahl von Linsen jeweils paarweise durch ein Paar von Linsen (9, 10, 20, 21) erfolgt.

5. Dentalkamera nach einem der Ansprüche 1 bis 4, worin die Verkippung der ersten lichtquellseitig angeordneten Linse (9) um einen Winkel (α) um eine Verkippungsachse (23) erfolgt, wobei die übrigen Linsen (10, 20, 21) ebenfalls um Verkippungsachsen (24, 25, 26) verkippt werden, die parallel zur Verkippungsachse (23) der ersten Linse (9) sind, wobei die Verkippungsachsen (23, 24, 25, 26) senkrecht zur Triangulationsebene (28) sind, die durch den Beleuchtungsstrahl (11) und den Beobachtungsstrahl (12) aufgespannt ist.

## Claims

1. A dental camera (1) with an objective lens (4) comprising at least two lenses (9, 10, 20, 21), wherein the dental camera (1) comprises a light source (3), a collector lens (60), an image sensor (7) and a telecentricity aperture (13) located in one focal plane of the objective lens (4), wherein an illuminating beam (11) emitted by the light source (3) is focused by the collector lens (60) by the light source (7) being reproduced by the collector lens (60) in the plane of an aperture stop (61) located in the focal plane of the objective lens (4) so that the focused image of the light source (3) is projected as homogeneously distributed light onto the plane of the measurement object (8) to be measured and is reflected back by the measurement object (8) as an observation beam (12), wherein the observation beam (12) penetrates the objective lens (4) and the telecentricity aperture (13) and is directed toward the image sensor (7), wherein the illuminating beam (11) is in part reflected by concave surfaces (9', 9'', 10', 10'', 20', 20'') of the lenses (9, 10, 20, 21) as reflection beams (17.1 to 17.8), wherein means (27) are present to tilt each of the lenses (9, 10, 20, 21) towards the illuminating beam (11) such that one optical axis (9''', 10''', 15.1, 15.2) of each lens (9, 10, 20, 21) has an angle of tilt (α) with respect to the illuminating beam (11) which is selected as large enough for the reflection beams (17.1 to 17.8) of the concave surfaces (9', 9'', 10', 10'', 20', 20'') to be reflected onto focal points (63.1 to 63.4) in a direction outside an aperture opening (13.1) of the telecentricity aperture (13), wherein at least one of the concave surfaces (9', 9', 9'', 10', 10'', 20', 20'') of the lenses (9, 10, 20) has a curvature such that the reflection beam is focused at a focal point (63.1 to 63.4) which lies in the plane of the telecentricity aperture (13) and outside the aperture opening (13.1) of the telecentricity aperture (13).

2. The dental camera according to claim 1, in which a second lens (10) is tilted by an angle of tilt (β) with respect to the illuminating beam (11), wherein the second lens (10) is arranged downstream of the first lens in the direction of the illuminating beam (11) and in which the aberrations caused by tilting the first lens (9) by an angle (α) are compensated by a second lens (10) so that the desired optical properties of the objective lens (4) are achieved.

3. The dental camera according to one of claims 1 to 2, in which the geometric parameters and material properties, such as radii, glass types, indices of refraction, distances and angles of tilt (α, β) of the lenses (9, 10, 20, 21) are selected such that the aberrations are adequately compensated in order to achieve the desired optical properties of the objective lens (4).

4. The dental camera according to one of claims 1 to 3, in which the compensation of aberrations in the case of an even number of lenses is respectively effected on a paired basis by a pair of lenses (9, 10, 20, 21).

5. The dental camera according to one of claims 1 to 4, in which the first lens (9) located at the light-source end is tilted around a tilt axis (23) by an angle (α), wherein the remaining lenses (10, 20, 21) are also tilted around tilt axes (24, 25, 26) which are parallel to the tilt axis (23) of the first lens (9), wherein the tilt axes (23, 24, 25, 26) are orthogonal to the triangulation plane (28) which is created by the illuminating beam (11) and the observation beam (12).

## Revendications

1. Caméra à usage dentaire (1) dotée d'un objectif (4) qui comprend au moins deux lentilles (9, 10, 20, 21), la caméra à usage dentaire (1) comprenant une source lumineuse (3), une lentille collectrice (60), un capteur d'image (7) et un diaphragme de télécentricité (13) disposé dans un plan focal de l'objectif (4), un faisceau d'éclairage (11) émis par la source lumineuse (3) étant concentré par la lentille collectrice (60), en ce que la source lumineuse (7) est représentée, à travers la lentille collectrice (60), dans le plan d'un diaphragme d'ouverture (61), qui se trouve dans le plan focal de l'objectif (4), de telle sorte que l'image concentrée de la source lumineuse (3) est représentée sous la forme d'une lumière répartie de manière homogène sur le plan de l'objet à mesurer (8) et est renvoyée comme faisceau d'observation (12) par l'objet de mesure (8), le faisceau d'observation (12) traversant l'objectif (4) et le diaphragme de télécentricité (13) et étant guidé sur le capteur d'image (7), le faisceau d'éclairage (11) étant réfléchi partiellement par des surfaces concaves (9', 9'', 10', 10", 20', 20'') des lentilles (9, 10, 20, 21) sous la forme de rayons de réflexion (17.1 à 17.8), des moyens (27) étant prévus pour basculer chacune des lentilles (9, 10, 20, 21) par rapport au faisceau d'éclairage (11) de telle manière qu'un axe optique (9"', 10''', 15.1, 15.2) de chaque lentille (9, 10, 20, 21) forme un angle de basculement (α) par rapport au faisceau d'éclairage (11), dont la grandeur est choisie de telle façon que les rayons de réflexion (17.1 à 17.8) des surfaces concaves (9', 9'', 10', 10'', 20', 20'') sont réfléchis sur des points focaux (63.1 à 63.4) dans une direction hors d'une ouverture de diaphragme (13.1) du diaphragme de télécentricité (13), au moins une surface concave (9', 9', 9", 10', 10", 20', 20") des lentilles (9, 10, 20) présentant une courbure telle que le faisceau de réflexion est concentré sur un point focal (63.1 à 63.4) qui se trouve dans le plan du diaphragme de télécentricité (13) et hors de l'ouverture de diaphragme (13.1) du diaphragme de télécentricité (13).

2. Caméra à usage dentaire selon la revendication 1, dans laquelle une deuxième lentille (10) est basculée selon un angle de basculement (β) par rapport au faisceau d'éclairage (11), la deuxième lentille (10) étant montée en aval de la première lentille dans le sens du faisceau d'éclairage (11) et que les aberrations provoquées par le basculement de la première lentille (9) selon un angle (α) sont compensées par une deuxième lentille (10), de telle sorte que les propriétés optiques souhaitées de l'objectif (4) sont obtenues.

3. Caméra à usage dentaire selon l'une des revendications 1 et 2, dans laquelle les paramètres géométriques et les propriétés du matériau, tels que les rayons, les types de verre, les indices de réfraction, les écarts et les angles de basculement (α, β) des lentilles (9, 10, 20, 21) sont choisis de telle manière que les aberrations sont compensées suffisamment pour obtenir les propriétés optiques souhaitées de l'objectif (4).

4. Caméra à usage dentaire selon l'une des revendications 1 à 3, dans laquelle, en cas de nombre pair de lentilles, la compensation des aberrations se fait respectivement par paire, par une paire de lentilles (9, 10, 20, 21).

5. Caméra à usage dentaire selon l'une des revendications 1 à 4, dans laquelle le basculement de la première lentille (9) disposée côté source lumineuse selon un angle (α) se fait autour d'un axe de basculement (23), les autres lentilles (10, 20, 21) étant également basculées autour des axes de basculement (24, 25, 26), qui sont parallèles à l'axe de basculement (23) de la première lentille (9), les axes de basculement (23, 24, 25, 26) étant perpendiculaires au plan de triangulation (28) qui est formé par le faisceau d'éclairage (11) et le faisceau d'observation (12).
